# EUROPÄISCHE PATENTSCHRIFT

(11) **EP 2 693 874 B1**
(45) Veröffentlichungstag und Bekanntmachung des Hinweises auf die Patenterteilung: **16.12.2015**
(21) Anmeldenummer: 12715329.4
(22) Anmeldetag: 05.04.2012
(51) Int. Cl.: A01N 25/30, A01N 31/02, A01N 37/44, A01P 1/00, C11D 1/10, A61L 2/18

(54) **DESINFEKTIONSMITTEL ZUR REINIGUNG VON BILDSCHIRMOBERFLÄCHEN ELEKTRONISCHER GERÄTE**
DISINFECTANT FOR CLEANING THE DISPLAYS OF ELECTRONIC DEVICES
DÉSINFECTANT POUR LE NETTOYAGE DES ÉCRANS DES DISPOSITIFS ÉLECTRONIQUES

(30) Priorität: 08.04.2011 DE 102011016452
(43) Veröffentlichungstag der Anmeldung: 12.02.2014
(73) Patentinhaber: Bode Chemie GmbH, 22525 Hamburg (DE)
(72) Erfinder: KRUG, Barbara, 20259 Hamburg (DE); MEYER, Stephanie, 25358 Horst (Holstein) (DE); BLOß, Richard, 25462 Rellingen (DE)
(74) Vertreter: Kossak, Sabine
(86) Internationale Anmeldenummer: PCT/EP2012/001519
(87) Internationale Veröffentlichungsnummer: WO 2012/136372

(56) Entgegenhaltungen:
- EP-A1- 1 468 699
- EP-A2- 1 967 066
- DE-A1- 19 801 821
- "Bacillol 30 Foam: Alcohol-based rapid disinfection", , 1. Februar 2011 (2011-02-01), Seiten 1-3, XP55029913, Gefunden im Internet: URL:http://www.bode-chemie.com/products/su rfaces/product_information/bacillol_30_foa m_int.pdf [gefunden am 2012-06-14]
- Davis, B.; Boittiaux, P.: "Amphoterics as surfactant biocides" In: "Comunicaciones presentadas a las 28 Jornadas del Comite Espanol de la Detergencia", 1998, XP009160292, Seiten 251-257, das ganze Dokument
- 'Bacillol AF', [Online] 29 September 2009, HAMBURG, GERMANY, Seiten 1 - 6, XP055162618 Gefunden im Internet: <URL:http://www.bexamed.de/downloads/bacill olaf.pdf> [gefunden am 2015-01-16]
- 'Bacillol 25 Safety Data Sheet', [Online] 09 Dezember 2008, Seiten 1 - 5, XP055029916 Gefunden im Internet: <URL:http://web.archive.org/web/20090106025 033/http://www.bode-chemie.com/sida/EN_R100 55.PDF> [gefunden am 2012-06-14]
- 'Bacillol 25', [Online] 01 Juli 2005, HAMBURG, GERMANY, Seiten 1 - 2, XP055162690 Gefunden im Internet: <URL:http://www.reinigungsberater.de/dokume nte/desinfektionsspray_bode_bacillol_25_5_l ,p-65923830,dl-produktblatt.pdf> [gefunden am 2015-01-16]
- 'Bacillol 25', [Online] 01 Januar 2008, Seiten 1 - 2, XP055162615 Gefunden im Internet: <URL:http://www.ramanweil.com/pdf/Bacillol_ 25.pdf> [gefunden am 2015-01-16]

## Beschreibung

Gegenstand der Erfindung ist die Verwendung eines Desinfektionsmittels, bestehend aus einer Mischung aus Ethanol, 1-Propanol und 2-Propanol, einem antimikrobiellen amphoteren Tensid und Wasser zur Desinfektion von Bildschirmoberflächen elektronischer Geräte.

Hygiene ist in der modernen Patientenversorgung ein Aspekt, der zunehmend an Wichtigkeit gewinnt. Im Fokus vieler Hygienemaßnahmen steht dabei die Desinfektion sowohl von Händen als auch von unbelebten Oberflächen. Dabei müssen insbesondere solche Oberflächen desinfiziert werden, die mit den Händen in Kontakt kommen, d.h. Handkontaktflächen. Patientennahe Oberflächen werden üblicherweise mit alkoholischen Desinfektionsmitteln behandelt. Diese Flächen und flächigen Gegenstände, wie beispielsweise Tische, Tabletts, Stühle, Türen, Türgriffe, Ablagen usw., sind dabei meist aus alkoholbeständigen Materialien hergestellt. Es handelt sich dabei um überwiegend "ortsfeste" Gegenstände, die einer routinemäßigen Reinigung und Desinfektion unterzogen werden.

Schwieriger ist die Desinfektion von Gegenständen, die nicht an einen Ort gebunden sind. Bei der Patientenversorgung werden beispielsweise zunehmend elektronische Geräte genutzt. Diese modernen Medien machen es möglich, dass Diagnosen, Laborergebnisse, digitale Darstellungen wie Röntgenbilder und dergleichen erfasst, gespeichert und übertragen werden. Dieses bedeutet mehr Sicherheit in der Behandlung, da es zu weniger Übertragungsfehlern kommt und die relevanten Informationen schnell abrufbar sind.

Nachteilig ist, dass diese Geräte von einem Patientenzimmer in das nächste gelangen können, ohne zwischendurch desinfiziert zu werden. Daher birgt die Nutzung dieser mobilen Kommunikationsgeräte ein erhöhtes Risiko bei der Übertragung von pathogenen Keimen. Die veröffentlichten Hygieneregeln besagen, dass vor und nach jedem Patientenkontakt die Hände desinfiziert werden müssen. Gelangen aber während der Behandlung Keime auf mobile Kommunikationsgeräte, werden diese nicht üblicherweise einer routinemäßigen Desinfektion unterzogen, da sie aus praktischen Gesichtspunkten meist in die Kitteltasche gesteckt werden, und somit zunächst nicht sichtbar sind. Es besteht daher die Gefahr, dass elektronische Geräte, insbesondere mobile Kommunikationsgeräte, bei der Desinfektion übersehen werden.

Entsprechende Geräte finden ihren Einsatz nicht nur in medizinischen und pflegerischen Einrichtungen, sondern verstärkt auch im OP-Bereich und auf Intensivstationen. Gerade in diesen sensiblen Hygienebereichen ist eine effektive Desinfektion aller Oberflächen notwendig, um eine Keimübertragung und daraus folgende Infektionen zu vermeiden.

Mobile Kommunikationsgeräte weisen häufig Oberflächen aus Kunststoff oder Glas, ggf. auch Komposit-Materialien mit einem dünnen mehrschichtigen Aufbau auf. Bei der Desinfektion unbelebter Oberflächen werden üblicherweise alkoholische Desinfektionsmittel eingesetzt. So ist aus der EP 1 020 115 A1 ein Desinfektionsmittel zur Schnelldesinfektion bekannt, das 68 -80 Gew.-% einer Mischung aus Monoalkoholen enthält. Dieses Desinfektionsmittel zeigt auch auf Grund des hohen Alkoholgehalts eine gute Wirksamkeit gegen Viren, Pilze und Bakterien.

Insbesondere Kunststoffoberflächen werden von organischen Lösungsmitteln wie Alkohol oder Aceton angelöst. Zudem enthalten viele Kunststoffe Weichmacher, die in organischen Lösungsmitteln löslich sind, und somit bei der Desinfektion ausgewaschen werden. Durch mehrfache Behandlung mit einem Desinfektionsmittel würde dem Material somit die Elastizität verloren gehen. Bei transparenten Kunststoffen ist dieser Effekt durch Trübung und Rissbildung zu erkennen.

Mobile Kommunikationsgeräte zeichnen sich durch multifunktionale Eigenschaften aus. Die sogenannten "Smartphones" sind über ein Touch-Display einfach zu bedienen. Die Eingabefläche ist technisch vergleichbar mit einer transparenten Folientastatur aus Polyesterfolie, die über ein Display, z.B. ein LED oder Flüssigkristalldisplay, gelegt wird.

Häufig werden die Displays mobiler Kommunikationsgeräte mit einer Display-Schutzfolie versehen, um diese vor Kratzern und anderen äußeren Einflüssen zu schützen. Diese Schutzfolien sind sehr kratz- und schlagfest und weisen dabei eine kristallklare Optik und angenehme Haptik bei uneingeschränkter Bedienbarkeit der Geräte auf. Diese Schutzfolien sind zumeist mehrschichtige Laminate, die die Eigenschaften der einzelnen Komponenten vereinen. Sie bestehen zumeist aus Acrylsäurepolymeren, Polycarbonat oder anderen bekannten Werkstoffen.

Bisher sind keine alkoholischen Desinfektionsmittel im Markt erhältlich, die zur Desinfektion von mobilen Kommunikationsgeräten, insbesondere zur Desinfektion der Bildschirme und Touch screens geeignet sind.

Bekannt sind Desinfektionsmittel zur Desinfektion unbelebter Oberflächen, die für bestimmte Oberflächen herkömmlicher Objekte und Geräte eine gute Materialverträglichkeit zeigen. Die EP 1 468 699 A1 offenbart die Verwendung eines Desinfektionsmittels auf Basis eines aliphatischen Monoalkohols und eines amphoteren Tensides in wässriger Lösung zur Abtötung von Mykobakterien. Dieses Desinfektionsmittel zeigt gegenüber Plexiglas eine gute Materialverträglichkeit. Die DE 198 01 821 A1 offenbart ein tuberkulozides Desinfektionsmittel mit einem N,N'-substituierten Glycin. Das Mittel zeigt eine gute tuberkulozide Wirksamkeit.

Alkoholische Desinfektionsmittel mit niedrigen Alkoholgehalten sind hingegen dafür bekannt, dass sie keine umfassende mikrobiozide und insbesondere keine fungizide Wirksamkeit aufweisen.

Zudem sind verschiedene alkoholhaltige Mittel zur Desinfektion von Oberflächen bekannt. Die EP 1 967 066 A2 offenbart ein Mittel zur Desinfektion unbelebter Oberflächen mit 35-70 Gew.-% einwertiger Alkohole in Kombination mit amphoteren Tensiden. Das Desinfektionsmittel zeigt eine gute Wirksamkeit gegen Bakterien, Pilze und Viren. Auf Grund des hohen Alkoholgehalts weist es jedoch keine ausreichende Materialverträglichkeit für empfindliche Kunststoffoberflächen, wie Plexiglas, auf.

Aufgabe der Erfindung ist es daher, ein Desinfektionsmittel bereitzustellen, das eine gute Materialverträglichkeit bei Kunststoffoberflächen elektronischer Geräte zeigt, insbesondere mobiler Geräte, die vorwiegend im medizinischen Bereich angewendet werden und einer professionellen Desinfektion bedürfen, insbesondere deren Bildschirmoberflächen wie Touch screens, und gleichzeitig eine gute mikrobiologische Wirksamkeit, insbesondere auch eine fungizide Wirksamkeit, aufweist. Das Desinfektionsmittel soll aufgrund der guten Materialverträglichkeit mit verschiedensten Werkstoffen universell und unabhängig von den Materialkomponenten und Materialeigenschaften einsetzbar sein, da davon auszugehen ist, dass nicht nur die transparente Displayfläche, sondern das gesamte Gehäuse der entsprechenden Geräte mit dem Desinfektionsmittel in Kontakt kommt und unterschiedliche Bauteile eines elektronischen Gerätes aus unterschiedlichen Kunststoffen bestehen.

Die Aufgabe wird erfindungsgemäß gelöst durch die Verwendung eines Desinfektionsmittels, enthaltend

| | |
|---|---|
| 25-32 Gew.-% | einer Mischung aus Ethanol, 1-Propanol und 2-Propanol, wobei mindestens 5,0 Gew.-% und höchstens 15 Gew.-% jeder einzelnen Alkoholkomponente enthalten sind, |
| 0,1-2,0 Gew.-% | eines antimikrobiellen, amphoteren Tensids; wobei das amphotere Tensid N-Alkylaminopropylglycin, mit Alkyl gleich C10 bis C16, ist und |
| | Wasser, |

zur Desinfektion von Bildschirmoberflächen elektronischer Geräte, wobei die desinfizierten Oberflächen zumindest teilweise aus temperaturbeständigen oder transparenten oder hochspiegelnden Kunststoffen oder Kunststoffbeschichtungen bestehen.

Weitere Ausführungsformen sind Gegenstand der Unteransprüche oder nachfolgend beschrieben.

Bevorzugt besteht das erfindungsgemäß verwendete Desinfektionsmittel aus

| | |
|---|---|
| 25-32 Gew.-% | einer Mischung aus Ethanol, 1-Propanol und 2-Propanol, wobei mindestens 5,0 Gew.-% und höchstens 15 Gew.-% jeder einzelnen Alkoholkomponente enthalten sind, |
| 0,1-2,0 Gew.-% | eines antimikrobiellen, amphoteren Tensids und |

Wasser, wobei sich die Komponenten zu 100 Gew.-% ergänzen.

Das erfindungsgemäß verwendete Desinfektionsmittel enthält bevorzugt 28 - 32 Gew.-% der Mischung aus Ethanol, 1-Propanol und 2-Propanol. Weiterhin enthält das Desinfektionsmittel bevorzugt 0,1 - 1,0 Gew.-% Tensid.

Das erfindungsgemäß verwendete Desinfektionsmittel enthält weiter bevorzugt
- 13 bis 15 Gew.-% Ethanol,
- 9 bis 10,5 Gew.-% 2-Propanol,
- 5 bis 6,5 Gew.-% 1-Propanol,
- 0,1 bis 2,0 Gew.-%, bevorzugt, 0,1 bis 1,0 Gew.-%, des antimikrobiellen amphoteren Tensids und
- Wasser, bevorzugt als Rest Wasser.

Dabei ist das verwendete antimikrobielle, amphotere Tensid N-Alkylaminopropylglycin, wobei der Alkylrest 10 bis 16 Kohlenstoffatome aufweist. Ein solches Tensid ist als 30-%ige wässrige Lösung unter dem Handelsnamen Rewocid WK 30 der Firma Evonik erhältlich.

Die entsprechend der erfindungsgemäßen Verwendung desinfizierten Bildschirmoberflächen elektronischer Geräte sind insbesondere die Bildschirmoberflächen von Touch screens, Mobiltelefonen, Smartphones, Flachbildschirmen, Laptopbildschirme, Tablet-PCs und PDAs, wobei insbesondere die Displays bzw. Touch screens oder die darauf applizierten Schutzfolien dieser Geräte desinfiziert werden. Die desinfizierten Oberflächen bestehen dabei zumindest teilweise aus temperaturbeständigen oder transparenten oder hochspiegelnden Kunststoffen oder Kunststoffbeschichtungen, vorzugsweise aus Polycarbonat oder Polysulfon. Ein bekanntes Polycarbonat ist beispielsweise Makrolon^{®} sowie ein bekanntes Polysulfon beispielsweise Tecason^{®}S ist.

Die erfindungsgemäße Verwendung des Desinfektionsmittels führt zur Abtötung von Hefen, insbesondere von *Candida albicans* und/oder zur Abtötung von Pilzen, insbesondere von *Aspergillus brasiliensis* (frühere Bezeichnung *Aspergillus niger*)*.* Dabei erfolgt bei der erfindungsgemäßen Verwendung des Desinfektionsmittels bevorzugt eine Log-4-Reduktion innerhalb von 0,5 Minuten gegenüber *Candida albicans* und/oder eine Log-4-Reduktion innerhalb von 1 Minute gegenüber *Candida albicans* gemäß "Standardmethoden der DGHM zur Prüfung chemischer Desinfektionsverfahren" (Stand 1. September 2001). Weiterhin wird durch die erfindungsgemäße Verwendung des Desinfektionsmittels bevorzugt mindestens eine Log-2-Reduktion innerhalb von 5 Minuten gegenüber *Apergillus brasiliensis* erreicht und/oder innerhalb von 10 Minuten mindestens eine Log-3-Reduktion gegenüber *Aspergillus brasiliensis* erreicht und/oder innerhalb von 15 Minuten eine Log-4-Reduktion gegenüber *Aspergillus brasiliensis* gemäß "Standardmethoden der DGHM zur Prüfung chemischer Desinfektionsverfahren" (Stand 1. September 2001) erreicht.

Das Verfahren "Standardmethoden der DGHM zur Prüfung chemischer Desinfektionsverfahren" (Stand 1. September 2001) ist dabei in der Durchführung und Auswertung äquivalent zu der internationalen EN 13727 ("Chemische Desinfektionsmittel und Antiseptika - Quantitativer Suspensionsversuch zur Prüfung der bakteriziden Wirkung chemischer Desinfektionsmittel für Instrumente im humanmedizinischen Bereich - Prüfverfahren und Anforderungen (Phase 2, Stufe 1); Deutsche Fassung EN 13727:2003). Der Prüfkeim *Aspergillus brasiliensis* ATCC 16404 entspricht dem Keim *Aspergillus nigerATCC* 16404 (frühere Bezeichnung) und ist diesem genetisch identisch.

Das erfindungsgemäß verwendete Desinfektionsmittel wird bevorzugt in Form eines Schaums, eines Sprühdesinfektionsmittels, einer Flüssigkeit oder in Form eines mit dem Desinfektionsmittel imprägnierten Tuchs, Schwamms oder anderer Applikationshilfe appliziert.

Die flüssige Zusammensetzung kann auf ein Tuch aufgebracht werden und in Form einer Wischdesinfektion verwendet werden. Sie kann aber auch versprüht werden, wobei die Zusammensetzung entweder als Flüssigkeit oder vorzugsweise mittels eines Dispensers mit Schaumapplikator als Schaum versprüht wird. Der Schaum kann direkt auf die zu reinigende Oberfläche appliziert oder mittels eines Tuches, eines Schwammes oder einer anderen Applikationshilfe aufgebracht werden. Die Zusammensetzung weist keine weiteren Inhaltsstoffe auf, insbesondere werden keine weiteren Komponenten zur Stabilisierung des Schaums benötigt.

Das erfindungsgemäß verwendete Desinfektionsmittel erlaubt unenivarteter weise trotz des niedrigen Alkoholgehaltes eine umfassende Abtötung von Hefen und Pilzen und zeigt somit eine sehr gute mikrobiologische und insbesondere fungizide Wirksamkeit. Die mikrobiologische Wirksamkeit insbesondere gegen Problemkeime wie Hefen und Pilze ist dabei deutlich höher als die der Einzelkomponenten und auf einen synergistischen Effekt der Alkoholmischung in Mischung mit dem amphoteren Tensid zurückzuführen. Ebenso überraschend ist die gute Wirksamkeit gegenüber Pilzen, wie Aspergillus brasiliensis. Auch hier war aufgrund der Wirksamkeiten der einzelnen Inhaltsstoffe nicht zu erwarten, dass bei einer so niedrigen Alkoholkonzentration eine gute Keimreduktion erreicht wird. Trotz des geringen Alkoholgehalts zeigt das erfindungsgemäß verwendete Desinfektionsmittel auch eine gute Wirksamkeit gegenüber schwer zu inaktivierenden unbehüllten Viren, wie z.B. Adeno-Viren.

Zudem ist bei erfindungsgemäßer Verwendung des Desinfektionsmittels eine gute Materialverträglichkeit gegeben, so dass das Desinfektionsmittel auch auf den empfindlichen Bildschirmoberflächen elektronischer Geräte wie z.B. Flachbildschirmen, Touch screens, PDAs, Mobiltelefonen, Tastaturen und insbesondere Kunststoffoberflächen mit Polysulfon oder auf den darauf applizierten Schutzfolien angewandt werden kann.

Die entsprechend der erfindungsgemäßen Verwendung desinfizierten Bildschirmoberflächen elektronischer Geräte sind insbesondere die Bildschirmoberflächen von Touchscreens (beispielsweise an Bedienterminals), Mobiltelefonen, Smartphones, zum Beispiel iPhone®, Blackberry®, Flachbildschirmen, Laptopbildschirmen, Tablet-PCs, Piepern und PDAs, wobei insbesondere die Displays bzw. Touchscreens oder die darauf applizierten Schutzfolien dieser Geräte desinfiziert werden. Die erfindungsgemäße Verwendung betrifft alle Flächen derartiger Geräte, die Kontakt mit den Händen und/oder anderen Hautoberflächen von Ärzten, Krankenschwestern, Pflegekräften und Patienten haben, insbesondere Handkontaktflächen.

Die Geräte befinden sich bevorzugt im Bereich klinischer und pflegerischer Einrichtungen, wie Behandlungsräumen, Stationen, Patientenzimmern, Untersuchungszimmern, Operationssälen, Kreißsälen, Ambulanzen, Intensivstationen in Pflegeeinrichtungen, Krankenhäusern und Arztpraxen, sowie beim mobilen Pflegedienst. Das erfindungsgemäß verwendete Desinfektionsmittel wird insbesondere in medizinischen und pflegerischen Einrichtungen, vorzugsweise im OP-Bereich und auf Intensiv-, Kranken- und Pflegestationen eingesetzt. Hier ist eine professionelle Desinfektion aller Oberflächen notwendig, um eine Keimübertragung und daraus folgende Infektionen zu vermeiden.

Die Desinfektion erfolgt dabei auf Oberflächen, die zumindest teilweise aus Kunststoffen bestehen. Wenn Kunststoffmaterialien unterhalb der Streckgrenze mechanisch gedehnt oder gespannt werden, kann es nach einem bestimmten Zeitraum zu Spannungsrissen im Material kommen. Die gleichzeitige Beanspruchung in Gegenwart einer veränderten chemischen Umgebung kann zu einer signifikanten Verkürzung dieses Zeitraumes führen. Dieses Phänomen wird als umgebungsbedingte Spannungsrissbildung (environmental stress cracking, ESC) bezeichnet.

Das erfindungsgemäß verwendete Desinfektionsmittel zeichnet sich besonders durch seine gute Materialverträglichkeit aus. Bevorzugt ist bei erfindungsgemäßer Verwendung des Desinfektionsmittels auf Polysulfon-Oberflächen die Materialverträglichkeit des Desinfektionsmittels gemessen als ESC-Potential Ω_{ESC} gemäß alternierender ESC-Prüfung < 1, vorzugsweise < 0,8 über eine Prüfdauer von 25 Minuten bei 25 °C. Bei erfindungsgemäßer Verwendung des Desinfektionsmittels auf Polysulfon-Oberflächen ist zudem bevorzugt die Spannungsrissbildung ΔΩ_{ESC} unter 5, vorzugsweise unter 4, weiter bevorzugt unter 3 gemäß ESC-Prüfung bei einer Oberflächentemperatur von 20°C bis 45°C.

Ein weiterer Aspekt, der bei der Desinfektion von Oberflächen eine Rolle spielt, sind mögliche durch das Präparat entstehende Rückstände. Das erfindungsgemäß verwendete Desinfektionsmittel ist ein Leave-on-Produkt, das nach der Desinfektion nicht entfernt werden braucht. Bei der Verwendung dürfen somit keine störenden Rückstände auf der Oberfläche verbleiben, die die Einsatzbereitschaft des elektronischen Gerätes beeinträchtigen würden. Zudem darf das Produkt nicht reizend sein, da die Hautoberfläche bei der Verwendung der Geräte mit der Bildschirmoberfläche bzw. Tastaturen in Berührung kommt und mögliche Rückstände des erfindungsgemäß verwendeten Desinfektionsmittels somit auf die Haut übertragen werden könnten. Es hat sich gezeigt, dass die auf der Oberfläche verbleibenden Rückstände des erfindungsgemäß verwendeten Desinfektionsmittels nicht reizend auf die Haut wirken, da die Alkohole nach der Anwendung verdunsten und nur geringe Mengen des amphoteren Tensids zurückbleiben und bevorzugt keine weiteren Inhaltsstoffe enthalten sind.

Das erfindungsgemäß verwendete Desinfektionsmittel zeichnet sich somit auch dadurch aus, dass die Zusammensetzung bevorzugt keine weiteren Alkohole, keine weiteren Wirkstoffe, keine quaternären Ammoniumverbindungen (QAV) und keine nichtionischen, anionischen oder kationischen Tenside enthält. Die erfindungsgemäße Verwendung zur Desinfektion von Bildschirmoberflächen kann selbstverständlich nicht nur im medizinischen und klinischen Bereich eingesetzt werden, sondern auch in anderen Bereichen. So ist die Desinfektion in öffentlichen Einrichtungen, insbesondere bei Automaten mit Touch screens, in der Lebensmittelindustrie, in Hotels, an Fahrkarten- oder Geldautomaten, öffentlichen Informationsterminals und vielen anderen Bereichen denkbar.

### Beispiele

Es wurde ein Desinfektionsmittel aus den in Tabelle 1 angegebenen Inhaltsstoffen hergestellt.

**Tabelle 1**

| | **Beispiel 1** |
|---|---|
| Aqua purificata | 69,50% |
| Ethanol | 14,00% |
| 2-Propanol | 10,00% |
| 1-Propanol | 6,00% |
| N-Alkylaminopropylglycin (Alkyl gleich C10 bis C16) | 0,50% |

Die Zusammensetzung weist einen pH-Wert von 8 auf.

Das Desinfektionsmittel gemäß Tabelle 1 wurde auf seine Wirksamkeit gegen Hefen und Pilze im Vergleich zu Lösungen der Einzelkomponenten gemäß Tabelle 2 getestet. Hierzu wurden folgende Vergleichslösungen angesetzt:

**Tabelle 2**

| | wässrige Lösung Alkohole | wässrige Lösung Tensid |
|---|---|---|
| Aqua purificata | 70,00% | 99,50% |
| Ethanol | 14,00% | --- |
| Isopropanol | 10,00% | --- |
| n-Propanol | 6,00% | --- |
| N-Alkylaminopropylglycin (Alkyl gleich C10 bis C16) | --- | 0,50% |

Es zeigen
- Figur 1 die mikrobiologischen Wirksamkeiten gegen Candida albicans (0,5 Minuten) und
- Figur 2 die mikrobiologischen Wirksamkeiten gegen Aspergillus brasiliensis (15 Minuten).

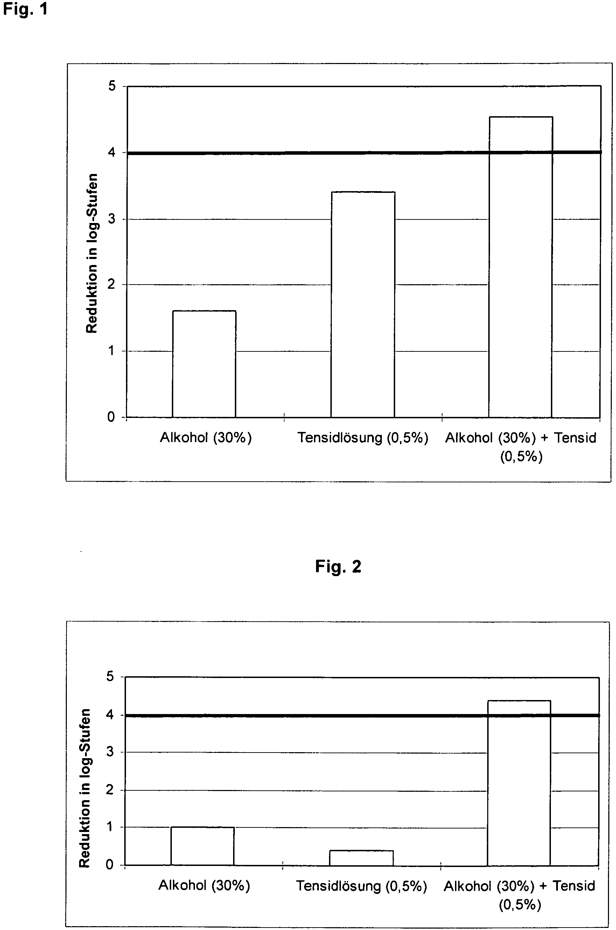

Figur 1 zeigt die mikrobiologischen Wirksamkeit einer wässrigen Alkoholmischung, einer reinen wässrigen Tensidlösung gemäß Tabelle 2 und der Desinfektionsmittelmischung gemäß Tabelle 1 gegenüber Candida albicans. Es ist zu erkennen, dass nur das erfindungsgemäß verwendete Desinfektionsmittel die geforderte Reduktion um 4 Log-Stufen erreicht. Die reine Alkoholmischung erzielt aufgrund des geringen Alkoholgehaltes keine ausreichende Reduktion der Hefen.

Wie in Figur 2 zu erkennen ist, addieren sich bei der Wirksamkeit gegenüber Pilzen nicht die Effekte der einzelnen Inhaltsstoffe, sondern es kommt zu einer synergistischen Wirksamkeit, durch die erst die geforderte Reduktion um 4 Log-Stufen erreicht wird.

### Prüfung Fungizidie

Die Bestimmung der fungiziden Wirkung im quantiativen Suspensionsversuch gemäß den "Standardmethoden der DGHM zur Prüfung chemischer Desinfektionsverfahren" (Stand 1. September 2001) beschreibt ein Prüfverfahren und die Mindestanforderungen an die fungizide Wirkung von chemischen Desinfektionsmitteln und antiseptischen Produkten zur Feststellung, ob ein Antiseptikum im Rahmen des im Anwendungsbereich beschriebenen Arbeitsgebiets und Feldes eine fungizide Wirkung aufweist oder nicht.

Die Produkte können nur in einer Konzentration von 80% oder darunter geprüft werden, da durch Zugabe der Prüfkeime und der Belastungssubstanz immer eine gewisse Verdünnung bewirkt wird.

Die Prüfkeime für eine Instrumentendesinfektion und Oberflächendesinfektion sind bei derfungiziden Wirksamkeit Candida albicans (ATCC 10231) und Aspergillus brasiliensis (ATCC 16404). Aspergillus brasiliensis wird in der Methode "Standardmethoden der DGHM zur Prüfung chemischer Desinfektionsverfahren" (Stand 1. September 2001) noch als Aspergillus niger bezeichnet.

Die Prüftemperatur beträgt 20°C, und zusätzlich 30°C bei einem Mittel zur Oberflächendesinfektion bzw. maximal 60°C bei einem Mittel zur Instrumentendesinfektion. Die Einwirkzeit beträgt 60 Minuten, für ein Mittel zur Oberflächendesinfektion werden zusätzlich 5 Minuten geprüft. Darüber hinaus werden Einwirkzeiten nach Herstellerangaben geprüft.

Produktprüflösungen sind in mindestens drei unterschiedlichen Konzentrationen in Wasser standardisierter Härte herzustellen, wobei eine Konzentration im wirksamen Bereich und eine Konzentration im nicht wirksamen Bereich liegen muss. Das Produkt im Lieferzustand darf als eine der Produktprüflösungen verwendet werden; in diesem Fall beträgt die höchste geprüfte Konzentration 80%.

Die Anzahl der Zellen in der Prüfsuspension wird mit dem Verdünnungsmittel auf 1,5*10⁸ KBE je ml bis 5,0*10⁸ KBE je ml eingestellt.

Die Belastungssubstanz muss nach den für das Produkt festgelegten Anwendungsbedingungen ausgewählt werden. Sie muss steril sein und mit einer zehnfach höheren Konzentration, als für die Prüfung benötigt wird, hergestellt werden. Bei geringer Belastung (clean condition) wird Rinderserumalbuminlösung in niedriger Konzentration (0,3 g/L) verwendet. Bei hoher Belastung (dirty condition) wird ein Gemisch von Rinderserumalbuminlösung in hoher Konzentration (3 g/L) mit gewaschenen Schaf-Erythrozyten (3mL/L) verwendet.

Zur Kontrolle wird statt einer Prüfsuspension eine Validierungssuspension verwendet, bei der die Prüfsuspension mit dem Verdünnungsmittel so verdünnt wird, dass sich 3,0*10² KBE je ml bis 1,6*10³ KBE je ml ergeben.

Es wurden 1 ml Belastungssubstanz und 1 ml Prüfsuspension in ein Reagenzglas pipettiert. Die Stoppuhr wurde sofort in Gang gesetzt, es wurde durchmischt und das Reagenzglas für 2 min +/- 10 s in ein auf 20°C eingestelltes Wasserbad gestellt. Nach Ablauf dieser Zeit wurden 8 ml einer der Produktprüflösungen zugegeben. Die Stoppuhr wurde bei Beginn der Zugabe wieder in Gang gesetzt, es wurde durchmischt und das Reagenzglas für die gewählte Einwirkzeit t in ein auf 20°C eingestelltes Wasserbad gestellt.

Unmittelbar vor dem Ende von t wurde die Lösung erneut durchmischt. Nach Ablauf von t wurde eine 1-ml-Probe des Prüfgemischs in ein Röhrchen mit 8,0 ml Neutralisationsmedium und 1 ml Wasser überführt. Es wurde durchmischt und in ein auf 20°C eingestelltes Wasserbad gestellt. Nach einer Neutralisationszeit von 5 min +/- 10 s wurde durchmischt und sofort eine Probe von 1 ml des neutralisierten Prüfgemischs (enthaltend Neutralisationsmedium, Produktprüflösung, Belastungssubstanz und Prüfsuspension) als Doppelbestimmung entnommen und im Oberflächenverfahren beimpft.

Zusätzlich wurden 0,5 ml dieses Gemischs in ein Röhrchen überführt, das 4,5 ml des Neutralisationsmediums enthielt, um das neutralisierte Prüfgemisch in einer Verdünnung von 10⁻¹ zu erhalten. Von jedem Verdünnungsröhrchen wurden Proben von 1 ml als Doppelbestimmung entnommen und im Oberflächenverfahren beimpft.

Bei der Verwendung des Oberflächenverfahrens wurde jede 1-ml-Probe, die in Anteile etwa gleicher Größe aufgeteilt ist, auf eine geeignete Anzahl (mindestens zwei) von auf der Oberfläche getrockneten Platten mit TSA ausgestrichen.

Die Platten wurden 20 h bis 24 h bebrütet und zur Bestimmung der Anzahl der koloniebildenden Einheiten (KBE) ausgezählt.

Das Ergebnis des quantiativen Suspensionsversuch unter hoher organischer Belastung bei Aspergillus brasiliensis ist in Tabelle 3 dargestellt.

**Tabelle 3**

| Prüfzeiten | Rezeptur gemäß Beispiel 1 | Rezeptur gemäß Vergleichsbeispiel 1 |
|---|---|---|
| | Reduktionsfaktor | Reduktionsfaktor |
| 5 min | 2,56 | 0,63 |
| 10 min | 3,58 | 0,80 |
| 15 min | 4,4 | 0,89 |

### Viruswirksamkeit

Für die Prüfung der Inaktivierung von Viren wurden (entsprechend der Leitlinie der DVV und des RKI) acht Volumenteile des Desinfektionsmittels mit einem Volumenanteil Virussuspension und einem Volumenanteil Aqua bidest. vermischt. Bei den Versuchen mit FKS-Belastung wurde anstelle von Aqua bidest. ein Volumenanteil FKS-Belastungssubstanz zugegeben. Des weiteren wurden neun Volumenanteile mit 0,1 Volumenanteil Virussuspension und 0,9 Volumenanteilen Aqua bidest. vermischt. Bei den Versuchen mit Belastung wurde an Stelle von Aqua bidest FKS-Belastungssubstanz zugegeben.

Alle Versuchsansätze wurden in geschlossenen Plastikröhrchen in einem Wasserbad bei 20 °C +/- 0,5 °C durchgeführt. Nach Ablauf der Einwirkzeiten wurden Teilmengen aus den Plastikröhrchen entnommen und die Restinfektiosität bestimmt. Die Bestimmung der Infektiosität erfolgte mit Hilfe einer Endpunkttitration im Mikrotiter-Verfahren. Dafür wurden die Proben nach ihrer Entnahme zunächst in ein eiskaltes Medium mit dem Faktor 10 verdünnt und jeweils 100 µL der Verdünnung in acht Kavitäten einer sterilen 96 well Mikrotiterplatte mit vorab vorgelegten CV-1-Zellen überführt (je 10 - 15 x 103 Zellen) und bei 37 °C im CO2-Brutschrank (5% CO2-Gehalt) inkubiert. Nach 21 Tagen erfolgte die Auswertung der Ansätze über die Beurteilung mit einem inversen Mikroskop.

Die Beurteilung der virusinaktivierenden Wirkung des zu prüfenden Desinfektionsmittels erfolgte durch Berechnung des Titerabfalls gegenüber den jeweils parallel durchgeführten Kontrolltitrationen ohne Desinfektionsmittel. Die Differenz wurde als Reduktionsfaktor mit einem Konfidenzintervall von 95 % angegeben.

In der Leitlinie werden die Begriffe "begrenzt viruzid" (wirksam gegen behüllte Viren) und "viruzid" (wirksam gegen unbehüllte Viren); im Sinne der Definition der Stellungnahme des Arbeitskreises Viruzidie am RKI verwendet. Die Wirksamkeit gegen unbehüllte Viren schließt eine Wirksamkeit gegen behüllte Viren ein.

Dabei sind die Prüfviren für den Wirkungsbereich "begrenzt viruzid" Vacciniavirus, Stamm Elstree3 und Bovine Viral Diarrhea Virus (BVDV), Stamm NADL. Die Prüfviren für den Wirkungsbereich "viruzid" sind Vacciniavirus, Stamm Elstree3, Poliovirus-Impfstamm Typ I, Stamm LSc-2ab, Adenovirus Typ 5, Stamm Adenoid 75 und Polyomavirus4 (SV 40), Stamm 777.

Die Viren sind in Zellkulturen oder anderen geeigneten Systemen zu vermehren. Die Methoden, die zur Herstellung der Virussuspension eingesetzt werden, können in Abhängigkeit vom Testvirus differieren. Der Gehalt der Suspensionen an Viren sollte nicht weniger als 108 TCID50/ml betragen.

Da das zu prüfende Desinfektionsmittel unverdünnt angewendet werden bzw. in gebrauchsfertiger Form in den Handel kommen soll, erfolgt die Prüfung infolge des notwendigen Zusatzes an Virussuspension und FKS-Belastung (fetales Kälberserum) bei einer Konzentration von 90 bzw. 80 % des Produktes in der Prüflösung.

Das Desinfektionsmittel gemäß Beispiel 1 zeigte eine gute Viruswirksamkeit innerhalb von maximal 5 Minuten mit einer Reduktion um mindestens 4 log-Stufen bei den Viren Vaccinia Virus, Bovine Viral Diarrhea Virus, Polyoma Virus und Rota Virus. So wurde für Vaccinia Virus und Bovine Viral Diarrhea Virus unter organischer Belastung (10% FKS) eine Reduktion des Virustiters um größer log 4 Stufen innerhalb von 30 Sekunden erreicht.

### Prüfung Materialverträglichkeit

Es wurde zudem die Materialverträglichkeit einer gemäß Beispiel 1 hergestellten Rezeptur geprüft.

Zur Prüfung und Beurteilung des Spannungsrissverhaltens (Environmental Stress Cracking, ESC) im Biegestreifenverfahren, wurde die Zusammensetzung gemäß Beispiel 1 gegenüber den Prüfmaterialien Plexiglas^{®} XT (PMMA, Polymethylmethacrylat, Evonik), Makrolon^{®} 281 (PC, Polycarbonat, Bayer) und Tecason^{®} S (PSU, Polysulfon, Ensinger) getestet.

Die Prüfungen wurden in Anlehnung an die DIN EN ISO 22088 Teil 1 und 3 (ehemalige DIN 53449 Teil 3 "Beurteilung des Spannungsrissverhaltens - Biegestreifenverfahren") im Klimaschrank mit Vliesbenetzung durchgeführt (DIN EN ISO 22088-1 Kunststoffe - Bestimmung der Beständigkeit gegen umgebungsbedingte Spannungsrissbildung (ESC) - Teil 1: Allgemeine Anleitung (ISO 22088-1:2006) und Teil 3: Biegestreifenverfahren (ISO 22088-3:2006)). Um eine zu rasch verlaufende Verdunstung des flüssigen Prüfproduktes zu verhindern, wurde ein inertes Vlies (Tecnojet A-650/1, Ahlstrom Milano) als Oberflächenauflage eingesetzt, welches gleichzeitig eine komplette Benetzung der Prüfkörperoberfläche gewährleistet. Als Hilfsmittel zur Auswertung erhaltener Prüfdaten kann das ESC-Potential Ω_{ESC} genutzt werden.

Für die Prüfung in Anlehnung an die DIN EN ISO 22088-1 wurden Kunststoffstreifen unter konstanter Biegezugspannung angeordnet und für eine zuvor festgelegte Zeitdauer einem Spannungsrisse bewirkenden Medium ausgesetzt. Mit einer Reihe von Schablonen mit abnehmenden Radien wurde in der äußeren Oberfläche eine zunehmend höhere Dehnung erzeugt. Nach festgelegter Beanspruchung durch das Spannungsrisse bewirkende Medium wurden die Probekörper entnommen, einer Sichtprüfung unterzogen und hinsichtlich der indikativen Eigenschaft, wie z. B. Zugfestigkeit, geprüft.

Die Herstellung der Probekörper erfolgt in Anlehnung an ISO 2818. Die Abmessungen der Probekörper sind 120 mm x 35 mm x 3,0 mm (Länge l x Breite b x Dicke d). Die Probekörper wurden vor dem Prüfen mindestens 24 h bei (23 ± 2) °C und (50 ± 5) % relativer Feuchte konditioniert. Die Prüftemperatur betrug 25 °C. Die Prüfung erfolgte mit einer alternierenden Prüffolge von 5 mal 5 min Kontakt zum Desinfektionsmittel und 5 min Ruhepause ohne Kontakt zum Desinfektionsmittel. Der Prüfzeitraum betrug 5, 10, 15, 20 und 25 Minuten. Die Biegeschablone wies einen Radius von 450 mm auf.

Die Biegezugdehnung ist der Nennwert der Dehnung in der auf Zug belasteten Oberfläche eines flachen Probekörpers der Dicke h, der über ein Kreissegment mit dem Radius r gebogen wird. Der Dehnungswert ist ein Wert aus einer Reihe von Dehnungswerten, die aufeinander folgenden Probekörpern während der Belastung auferlegt werden. Das ESC-Potential Ω_{ESC} ist das Verhältnis des Wertes der im Prüfmedium bestimmten Bruchdehnung zu der bei gleicher Belastungsdauer im Referenzmedium (üblicherweise Luft) bestimmten Bruchdehnung. Bei einem Wert von > 1 liegt bei gegebenen Prüfbedingungen eine Tendenz zur Spannungsrissbildung vor.

Bei den durchgeführten Spannungsrissprüfungen wurden mit dem Kunststoffmaterial Polysulfon Prüfspannungen von 9,13 MPa realisiert.

Bei dem Vergleich der Einzelprüfungen gegenüber den alternierenden Prüffolgen ist bei der 450 mm Ringschablone zu erkennen, dass die alternierende Prüffolge gegenüber den Einzelprüfungen intensivere Beeinflussungen aufzeigen. Die alternierende Prüffolge wurde in einem vergleichenden Versuch bei unterschiedlicher chemischer Umgebung eingesetzt. Es wurden dabei die Rezeptur gemäß Beispiel 1, sowie ein Flächendesinfektionsmittel gemäß Vergleichsbeispiel 1. Das Ergebnis dieser Untersuchung ergab für die Rezeptur gemäß Beispiel 1 die geringste Tendenz zur Bildung von Spannungsrissen, die bei den erweiterten Prüfungen bis 25 Minuten immer noch deutlich unterhalb des Grenzwertes von 1 lagen.

Das Ergebnis der Prüfung bei 25 °C mit dem Kunststoffmaterial Polysulfon in einer alternierenden Prüfung (5 min Einwirkzeit, 5 min Erholungsphase, 5 min Einwirkzeit, 5 min Erholungsphase, usw.) ist in Tabelle 4 dargestellt.

**Tabelle 4**

| Einwirkzeit | Rezeptur gemäß Beispiel 1 | Rezeptur gemäß Vergleichsbeispiel 1 |
|---|---|---|
| | ESC-Potential Ω_{ESC} | ESC-Potential Ω_{ESC} |
| 5 min | 0,28 | 0 |
| 10 min | 0,31 | 0,31 |
| 15 min | 0,31 | 0,56 |
| 20 min | 0,47 | 0,81 |
| 25 min | 0,56 | 1,13 |

Während der Einwirkzeit von 5 Minuten wurde der Prüfkörper mit dem Produkt feucht gehalten. In der Ruhephase von 5 Minuten trocknete der Prüfkörper.

Bei einem ESC-Potential Ω_{ESC} > 1 liegt unter diesen Prüfbedingungen eine Tendenz zur Spannungsrissbildung vor. Hier wird deutlich, dass mit der Rezeptur gemäß Vergleichsbeispiel 1 behandelte Prüfkörper die Neigung zum Schadensaufbau haben, indem bei diesem Muster nach kurzer Zeit das ESC-Potential Ω_{ESC} den Wert 1 überschreitet.

Tabelle 5 zeigt das Ergebnis der Prüfung bei 25 °C bis 45 °C mit dem Kunststoffmaterial Polysulfon unter Betrachtung der Differenzen der ESC-Potentiale ΔΩ_{ESC} bei den jeweiligen Temperaturen, so können mit Δt = konstant (10 Minuten, Anstieg von Ω_{ESC} über die Zeit von 5 bis 15 Minuten) folgende Abhängigkeit aufgezeigt werden.

**Tabelle 5**

| Temperatur | Rezeptur gemäß Beispiel 1 | Rezeptur gemäß Vergleichsbeispiel 1 |
|---|---|---|
| | ΔΩ_{ESC} | ΔΩ_{ESC} |
| 25 °C | 0,4 | 0,4 |
| 35 °C | 0,8 | 1,6 |
| 45 °C | 2,3 | 5,7 |

Die ausgeprägteste Temperaturabhängigkeit zeigt die Probe der Vergleichsbeispiel 1, bei der knapp unterhalb von 35°C Wechselwirkungsreaktionen einsetzen, die gegenüber der Rezeptur gemäß Beispiel 1 nicht vorhanden sind, oder noch nicht eingesetzt haben.

Zudem wurde eine Reihe von Untersuchungen zur Verträglichkeit verschiedener Materialien mit dem Desinfektionsmittel durchgeführt. Zu diesem Zweck wurden Prüfkörper aus verschiedenen Materialien über einen Zeitraum von 4 Wochen in das gebrauchsfertige Desinfektionsmittel eingelegt und in regelmäßigen Abständen optisch begutachtet.

Zur Bestimmung der Materialverträglichkeit wurden standardisierte Prüfkörper aus verschiedenen Materialien verwendet (6 x 2 cm bei einer Schichtdicke je nach Material von 1 bis 3 mm):
Metalle: Edelstahl, Aluminium, Kupfer, Messing
Kunststoffe: Gummi (Kautschuk), Polysulfon, Latex, PVC, Polymethylmethacrylat (Plexiglas^{®}), Silikon, Polystyrol, Polytetrafluorethylen (Teflon^{®}), Polycarbonat (Makrolon^{®}), Vinylidenfluorid-Hexafluorpropylen-Copolymerisat (Viton^{®}), Polyethylen, Polypropylen, Weichgummi (Butadien - Kautschuk)

Zur Vorbereitung wurden die Prüfkörper mit einem Neutralreiniger gründlich gesäubert, mit Wasser gespült und dann luftgetrocknet. Die Prüfkörper wurden dann einzeln in die gebrauchsfertige Desinfektionslösung in Schraubdeckelgläsern bei Raumtemperatur eingelegt. Die Begutachtung der Materialien erfolgte optisch nach: 1. Tag, 1. Woche, 2. Woche, 3. Woche, 4. Woche.

Bei allen getesteten Metallen trat über den kompletten Zeitraum von 4 Wochen keine Korrosion auf. Bei allen getesteten Kunststoffen trat keine Materialschädigung ein. Unter den durchgeführten Testbedingungen ist die erfindungsgemäß verwendete Desinfektionslösung nicht korrosiv und nicht materialschädigend und kann daher als verträglich mit den oben genannten Materialien angesehen werden.

In einem praxisnahen Anwendungstest wurden zudem unterschiedliche Gegenstände aus verschiedenen Materialien über einen längeren Zeitraum regelmäßig mit der Desinfektionslösung behandelt. Die Gegenstände waren Flachbildschirme/ Monitore/ Touchscreens, Tastaturen, Laptops, Mobiltelefone und schnurlose Haustelefone. Dabei wurde jeder Gegenstand mindestens zehn mal mit dem Desinfektionsmittel behandelt.

Für die praxisnahe Durchführung wurde auf ein sauberes Einmaltuch eine ausreichende Menge des Desinfektionsmittels gegeben. Der auf das Tuch applizierte Schaum wurde auf die zu desinfizierende Fläche aufgetragen und verteilt, so dass die Oberfläche vollständig mit einem gleichmäßigen Flüssigkeitsfilm benetzt war. Bei unebenen Oberflächen wie z. B. Tastaturen eignete sich die Anwendung als Schaum im besonderen Maße, da hierdurch vermieden wird, dass zuviel Flüssigkeit in die Zwischenräume gelangte. Die Oberfläche wurde während der gesamten Einwirkzeit feucht gehalten.

Bei keinem der Gegenstände wurde eine optische oder funktionelle Veränderung der Materialien festgestellt. Es kam weder zu Trübungen oder Rissbildungen, noch zu Ablösungen von (Tastatur-)Beschriftungen oder anderen Effekten, die auf die Verwendung des Desinfektionsmittels zurückzuführen sind. Auch nach mehrmaliger Anwendung konnte keine Filmbildung durch Inhaltsstoffe der Desinfektionslösung auf den Testoberflächen festgestellt werden.

## Patentansprüche

1. Verwendung eines Desinfektionsmittels, enthaltend
25-32 Gew.-% einer Mischung aus Ethanol, 1-Propanol und 2-Propanol, wobei mindestens 5,0 Gew.-% und höchstens 15 Gew.-% jeder einzelnen Alkoholkomponente enthalten sind,
0,1-2,0 Gew.-% eines antimikrobiellen, amphoteren Tensids, wobei das amphotere Tensid N-Alkylaminopropylglycin, mit Alkyl gleich C10 bis C16, ist und
Wasser
zur Desinfektion von Bildschirmoberflächen elektronischer Geräte, wobei die desinfizierten Oberflächen zumindest teilweise aus temperaturbeständigen oder transparenten oder hochspiegelnden Kunststoffen oder Kunststoffbeschichtungen bestehen.

2. Verwendung nach Anspruch 1, **dadurch gekennzeichnet, dass** das Desinfektionsmittel besteht aus
| | |
|---|---|
| 25-32 Gew.-% | der Mischung aus Ethanol, 1-Propanol und 2-Propanol, wobei mindestens 5,0 Gew.-% und höchstens 15 Gew.-% jeder einzelnen Alkoholkomponente enthalten sind, |
| 0,1-2,0 Gew.-% | des antimikrobiellen, amphoteren Tensids und |
| 66 - 71,9 Gew.-% | Wasser, |
wobei sich die Komponenten zu 100 Gew.-% ergänzen.

3. Verwendung nach Anspruch 1 oder 2, **dadurch gekennzeichnet, dass** das Desinfektionsmittel als Mischung aus Ethanol, 1-Propanol und 2-Propanol,
13-15 Gew.-% Ethanol,
9-10,5 Gew.-% 2-Propanol,
5-6,5 Gew.-% 1-Propanol,
enthält.

4. Verwendung gemäß einem der vorherigen Ansprüche, **dadurch gekennzeichnet, dass** die elektronischen Geräte Flachbildschirme, Laptopbildschirme, Touch screens, Displays, Displays von Mobiltelefonen, Smart Phones, Tablet PCs oder PDAs sind, insbesondere Geräte in medizinischen und pflegerischen Einrichtungen, vorzugsweise im OP-Bereich und auf Intensiv-, Kranken- und Pflegestationen.

5. Verwendung gemäß einem der vorherigen Ansprüche, **dadurch gekennzeichnet, dass** die desinfizierten Oberflächen aus Polycarbonat und/oder Polyacrylat und/oder Polysulfon bestehen.

6. Verwendung gemäß einem der vorherigen Ansprüche, **dadurch gekennzeichnet, dass** auf Polysulfon-Oberflächen die Materialverträglichkeit des Desinfektionsmittels gemessen als ESC-Potential Ω_{ESC} gemäß alternierender ESC-Prüfung kleiner 1, vorzugsweise kleiner 0,8 ist über eine Prüfdauer von 25 Minuten bei 25 °C.

7. Verwendung gemäß einem der vorherigen Ansprüche, **dadurch gekennzeichnet, dass** auf Polysulfon-Oberflächen bei einer Oberflächentemperatur von 20 °C bis 45 °C die Spannungsrissbildungsneigung ΔΩ_{ESC} unter 5 vorzugsweise unter 4, weiter bevorzugt unter 3 liegt gemäß ESC-Prüfung.

8. Verwendung gemäß einem der vorherigen Ansprüche, **dadurch gekennzeichnet, dass** die Desinfektion eine Abtötung von Hefen, insbesondere von Candida albicans, und/oder eine Abtötung von Pilzen, insbesondere von Aspergillus brasiliensis, umfasst.

9. Verwendung gemäß einem der vorherigen Ansprüche, **dadurch gekennzeichnet, dass** das Desinfektionsmittel innerhalb von 0,5 Minuten eine log 4 Reduktion gegenüber Candida albicans gemäß den "Standardmethoden der DGHM zur Prüfung chemischer Desinfektionsverfahren" (Stand 1. September 2001) im quantitativen Suspensionsversuch bewirkt.

10. Verwendung gemäß einem der vorherigen Ansprüche, **dadurch gekennzeichnet, dass** das Desinfektionsmittel innerhalb von 15 Minuten eine log 4 Reduktion gegenüber Aspergillus brasiliensis gemäß den "Standardmethoden der DGHM zur Prüfung chemischer Desinfektionsverfahren" (Stand 1. September 2001) im quantitativen Suspensionsversuch bewirkt.

11. Verwendung gemäß einem der vorherigen Ansprüche, **dadurch gekennzeichnet, dass** das Desinfektionsmittel in Form eines Schaums, eines Sprühdesinfektionsmittels, einer Flüssigkeit oder eines mit dem Desinfektionsmittel imprägnierten Tuchs, Schwamms oder einer mit dem Desinfektionsmittel imprägnierten anderen Applikationshilfe appliziert wird.

12. Verwendung gemäß einem der vorherigen Ansprüche, **dadurch gekennzeichnet, dass** das Desinfektionsmittel in Form eines Leave-on-Produktes appliziert wird.

## Claims

1. Use of a disinfectant, containing
| | |
|---|---|
| 25-32 wt.% | of a mixture made of ethanol, 1-propanol, and 2-propanol, wherein at least 5.0 wt.% and at most 15 wt.% of each individual alcohol component is contained, |
| 0.1-2.0 wt.% | of an antimicrobial amphoteric surfactant, wherein the amphoteric surfactant is n-alkyl aminopropyl glycine, with the same alkyl from C10 to C16, and |
| | water |
for disinfecting the displays of electronic devices, wherein the disinfected surfaces consist at least partially of temperature-resistant or transparent or highly-reflective plastics or plastic coatings.

2. Use according to Claim 1, **characterized in that** the disinfectant consists of
| | |
|---|---|
| 25-32 wt.% | of the mixture made of ethanol, 1-propanol, and 2-propanol, wherein at least 5.0 wt.% and at most 15 wt.% of each individual alcohol component is contained, |
| 0.1-2.0 wt.% | of an antimicrobial, amphoteric surfactant, and |
| 66-71.9 wt.% | of water. |
wherein the components add up to 100 wt.%.

3. Use according to Claim 1 or 2, **characterized in that** the disinfectant contains 13-15 wt.% of ethanol,
9-10.5 wt.% of 2-propanol,
5-6.5 wt.% of 1-propanol
as the mixture made of ethanol, 1-propanol, and 2-propanol.

4. Use according to one of the preceding claims, **characterized in that** the electronic devices are flat-panel displays, laptop screens, touch screens, displays, displays on mobile telephones, smartphones, tablet PCs, or PDAs, in particular devices in medical and nursing facilities, preferably in the surgical areas and in intensive care units, hospital wards, and nursing stations.

5. Use according to one of the preceding claims, **characterized in that** the disinfected surfaces consist of polycarbonate and/or polyacrylate and/or polysulfone.

6. Use according to one of the preceding claims, **characterized in that**, on polysulfone surfaces, the compatibility of materials of the disinfectant, measured as the environmental stress cracking potential Ω_{ESC} according to alternating ESC test, is less than 1, preferably less than 0.8, over a test duration of 25 minutes at 25°C.

7. Use according to one of the preceding claims, **characterized in that**, on polysulfone surfaces at a surface temperature from 20°C to 45°C, the stress cracking tendency ΔΩ_{ESC} is less than 5, preferably less than 4, more preferably less than 3, according to ESC testing.

8. Use according to one of the preceding claims, **characterized in that** the disinfection includes a killing of yeasts, in particular Candida albicans, and/or a killing of fungi, in particular Aspergillus brasiliensis.

9. Use according to one of the preceding claims, **characterized in that** the disinfectant causes a log 4 reduction with respect to Candida albicans within 0.5 minutes in the quantitative suspension test according to the "Standard Methods of the DGHM for Testing Chemical Disinfection Procedures" (as of 1 September 2001).

10. Use according to one of the preceding claims, **characterized in that** the disinfectant causes a log 4 reduction with respect to Aspergillus brasiliensis within 15 minutes in the quantitative suspension test according to the "Standard Methods of the DGHM for Testing Chemical Disinfection Procedures" (as of 1 September 2001).

11. Use according to one of the preceding claims, **characterized in that** the disinfectant is applied in the form of a foam, a spray disinfectant, a liquid, or a cloth or sponge or another application aid impregnated with the disinfectant.

12. Use according to one of the preceding claims, **characterized in that** the disinfectant is applied in the form of a leave-on product.

## Revendications

1. Utilisation d'un agent désinfectant comprenant
- de 25 à 32 % en poids d'un mélange d'éthanol, de propan-1-ol et de propan-2-ol, étant entendu que sont contenus là-dedans au moins 5,0 % en poids et au plus 15 % en poids de chaque composant alcool pris individuellement,
- de 0,1 à 2,0 % en poids d'un tensio-actif amphotère antimicrobien, étant entendu que ce tensio-actif amphotère est une N-(alkylamino-propyl)-glycine comportant un groupe alkyle de 10 à 16 atomes de carbone,
- et de l'eau,
pour désinfecter des surfaces d'écrans d'appareils électroniques, étant entendu que les surfaces désinfectées sont constituées, au moins en partie, de matériaux artificiels ou de couches de matériaux artificiels thermiquement stables, transparents ou hautement réfléchissants.

2. Utilisation conforme à la revendication 1, **caractérisée en ce que** l'agent désinfectant est constitué
- de 25 à 32 % en poids d'un mélange d'éthanol, de propan-1-ol et de propan-2-ol, étant entendu que sont contenus là-dedans au moins 5,0 % en poids et au plus 15 % en poids de chaque composant alcool pris individuellement,
- de 0,1 à 2,0 % en poids du tensio-actif amphotère antimicrobien,
- et de 66 à 71,9 % en poids d'eau,
étant entendu que le total des composants fait 100 % en poids.

3. Utilisation conforme à la revendication 1 ou 2, **caractérisée en ce que** l'agent désinfectant contient, en tant que mélange d'éthanol, de propan-1-ol et de propan-2-ol, 13 à 15 % en poids d'éthanol, 9 à 10,5 % en poids de propan-2-ol et 5 à 6,5 % en poids de propan-1-ol.

4. Utilisation conforme à l'une des revendications précédentes, **caractérisée en ce que** les appareils électroniques sont des écrans plats, des écrans d'ordinateur portable, des écrans tactiles, des afficheurs, des écrans de téléphone mobile, des smartphones, des PC tablettes ou des assistants numériques personnels, en particulier des appareils de dispositifs médicaux ou infirmiers, de préférence situés dans une salle d'opération, un service de soins intensifs, une infirmerie ou un dispensaire

5. Utilisation conforme à l'une des revendications précédentes, **caractérisée en ce que** les surfaces désinfectées sont en polycarbonate et/ou en polyacrylate et/ou en polysulfone.

6. Utilisation conforme à l'une des revendications précédentes, **caractérisée en ce que**, sur les surfaces en polysulfone, la compatibilité de l'agent désinfectant avec ce matériau, évaluée par le potentiel de fissuration sous contrainte environnementale, Ω_{FCE}, lors d'un essai de FCE alternée, est inférieure à 1, de préférence inférieure à 0,8, pour un essai durant 25 minutes à 25 °C.

7. Utilisation conforme à l'une des revendications précédentes, **caractérisée en ce que**, sur les surfaces en polysulfone et pour une température de surface de 20 à 45 °C, la tendance à la fissuration sous contrainte, ΔΩ_{FCE}, vaut moins de 5, de préférence moins de 4 et mieux encore moins de 3, d'après un essai de FCE.

8. Utilisation conforme à l'une des revendications précédentes, **caractérisée en ce que** la désinfection comporte le fait d'éliminer des levures, en particulier Candida albicans, et/ou le fait d'éliminer des champignons, en particulier Aspergillus brasiliensis.

9. Utilisation conforme à l'une des revendications précédentes, **caractérisée en ce que** l'agent désinfectant agit contre Candida albicans en causant une réduction de log 4 en l'espace de 0,5 minute, d'après les « Méthodes normalisées de la SAHM (Société Allemande d'hygiène et de microbiologie) pour les tests de procédés chimiques de désinfection » (état du 1^{er} septembre 2001), dans un essai quantitatif sur suspension.

10. Utilisation conforme à l'une des revendications précédentes, **caractérisée en ce que** l'agent désinfectant agit contre Aspergillus brasiliensis en causant une réduction de log 4 en l'espace de 15 minutes, d'après les « Méthodes normalisées de la SAHM (Société Allemande d'hygiène et de microbiologie) pour les tests de procédés chimiques de désinfection » (état du 1^{er} septembre 2001), dans un essai quantitatif sur suspension.

11. Utilisation conforme à l'une des revendications précédentes, **caractérisée en ce que** l'agent désinfectant est appliqué sous la forme d'une mousse, d'un agent désinfectant en pulvérisateur, d'un liquide, ou d'un linge ou d'une éponge imprégné(e) de l'agent désinfectant, ou encore d'un applicateur d'une autre nature, imprégné de l'agent désinfectant.

12. Utilisation conforme à l'une des revendications précédentes, **caractérisée en ce que** l'agent désinfectant est appliqué sous la forme d'un produit sans rinçage.
